(19) Europäisches Patentamt
European Patent Office
Office européen des brevets

(11) Veröffentlichungsnummer: **0 094 539**
**B1**

(12) **EUROPÄISCHE PATENTSCHRIFT**

(45) Veröffentlichungstag der Patentschrift: **21.08.85**

(51) Int. Cl.⁴: **C 07 C 103/375**, A 01 N 37/18

(21) Anmeldenummer: **83104270.0**

(22) Anmeldetag: **02.05.83**

(54) **Halogenpropargylformamide.**

(30) Priorität: **18.05.82 DE 3218611**
**02.10.82 DE 3236522**

(43) Veröffentlichungstag der Anmeldung:
**23.11.83 Patentblatt 83/47**

(45) Bekanntmachung des Hinweises auf die Patenterteilung:
**21.08.85 Patentblatt 85/34**

(84) Benannte Vertragsstaaten:
**AT BE CH DE FR GB IT LI NL SE**

(56) Entgegenhaltungen:
**DE - A - 2 251 205**
**DE - A - 2 919 196**

(73) Patentinhaber: **BAYER AG, Konzernverwaltung RP Patentabteilung, D-5090 Leverkusen 1 Bayerwerk (DE)**

(72) Erfinder: **Oeckl, Siegfried, Dr., Auf der Höhe 74, D-5060 Bergisch-Gladbach (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen (DE)**
Erfinder: **Kuck, Karl-Heinz, Dr., Heerstrasse 24, D-4018 Langenfeld (DE)**
Erfinder: **Paulus, Wilfried, Dr., Deswatinesstrasse 90, D-4150 Krefeld (DE)**
Erfinder: **Genth, Hermann, Dr., Am Heckerhof 60, D-4150 Krefeld (DE)**

EP 0 094 539 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Halogenpropargylformamide, ein Verfahren zu ihrer Herstellung sowie ihre Verwendung als Schädlingsbekämpfungsmittel.

Seit langem sind Schwermetallsalze der Ethylen-1,2-bis-(dithiocarbaminsäure), insbesondere das Zink-ethylen-1,2-bis-(dithiocarbamidat), in Landwirtschaft und Gartenbau zur Bekämpfung von pflanzenpathogenen Pilzen in Gebrauch (vgl. R. Wegler, »Chemie der Pflanzenschutz- und Schädlingsbekämpfungsmittel«, Band 2, Seite 65, Springer-Verlag Berlin/Heidelberg/New York (1970).

Weiterhin ist seit langem bekannt, daß N-Trihalogenmethylthiogruppen enthaltende Verbindungen als Fungizide in Landwirtschaft und Gartenbau verwendet werden. So wird z. B. N-(Trichlormethylthio)-tetrahydrophthalimid im Obst- und Weinbau zur Bekämpfung von Pilzkrankheiten praktisch eingesetzt (vgl. DE-PS 887 506 und Angew. Chem. 76, 807 (1964)).

Weiterhin sind anorganische Kupfer-Verbindungen bekannt, die ein breites fungizides Wirkungsspektrum aufweisen (vg. K.H. Büchel, »Pflanzenschutz und Schädlingsbekämpfung«, Seiten 121 und 122, Georg Thieme Verlag Stuttgart). Außerdem sind Jodpropargyl-Verbindungen, wie das N-Butyljodpropargylcarbamat, als Anstrichfungizide (vgl. DE-OS 3 116 653) bekannt.

Es wurden neue Halogenpropargylformamide der Formel (I) gefunden

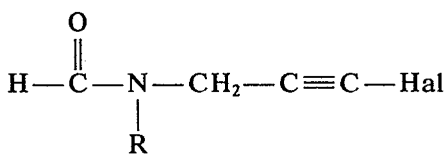

(I)

in welcher

R für Wasserstoff, Alkyl mit 1 bis 20 Kohlenstoffatomen, Halogenalkyl mit 1 bis 5 Halogenatomen und 1 bis 8 Kohlenstoffatomen, für Cycloalkyl mit 5 bis 10 Kohlenstoffatomen, für Alkenyl mit 2 bis 6 Kohlenstoffatomen, für Alkinyl mit 2 bis 6 Kohlenstoffatomen, für Alkinyl mit 2 bis 6 Kohlenstoffatomen, für Halogenalkinyl mit 1 bis 3 Halogen- und 2 bis 6 Kohlenstoffatomen, für gegebenenfalls durch Alkyl mit 1 bis 6 Kohlenstoffatomen und Halogen gleich oder verschieden, ein- bis fünffach im Arylteil substituiertes Aralkyl mit 1 bis 6 Kohlenstoffatomen im Alkylrest und 6 bis 10 Kohlenstoffatomen im Arylrest, für gegebenenfalls ein- bis fünffach, gleich oder verschieden durch Alkyl mit 1 bis 6 Kohlenstoffatomen, Halogen, Nitro, Alkoxy mit 1 bis 6 Kohlenstoffatomen, Halogenalkyl mit 1 bis 5 Halogen- und 1 bis 6 Kohlenstoffatomen, und Halogenalkylthio mit 1 bis 5 Halogen- und 1 bis 6 Kohlenstoffatomen substituiertes Aryl und

Hal für Jod oder Brom stehen.

Man erhält die neuen Halogenpropargylformamide der Formel (I)

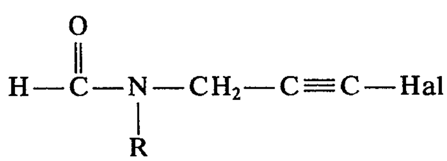

(I)

in welcher

R für Wasserstoff, Alkyl mit 1 bis 20 Kohlenstoffatomen, Halogenalkyl mit 1 bis 5 Halogenatomen und 1 bis 8 Kohlenstoffatomen, für Cycloalkyl mit 5 bis 10 Kohlenstoffatomen, für Alkenyl mit 2 bis 6 Kohlenstoffatomen, für Alkinyl mit 2 bis 6 Kohlenstoffatomen, für Halogenalkinyl mit 1 bis 3 Halogen und 2 bis 6 Kohlenstoffatomen, für gegebenenfalls durch Alkyl mit 1 bis 6 Kohlenstoffatomen und Halogen gleich oder verschieden, ein- bis fünffach im Arylteil substituiertes Aralkyl mit 1 bis 6 Kohlenstoffatomen im Alkylrest und 6 bis 10 Kohlenstoffatomen im Arylrest, für gegebenenfalls ein- bis fünffach, gleich oder verschieden durch Alkyl mit 1 bis 6 Kohlenstoffatomen, Halogen, Nitro, Alkoxy mit 1 bis 6 Kohlenstoffatomen, Halogenalkyl mit 1 bis 5 Halogen- und 1 bis 6 Kohlenstoffatomen, und Halogenalkylthio mit 1 bis 5 Halogen- und 1 bis 6 Kohlenstoffatomen substituiertes Aryl und

Hal für Jod oder Brom stehen,

wenn man Propargylformamide der Formel (II)

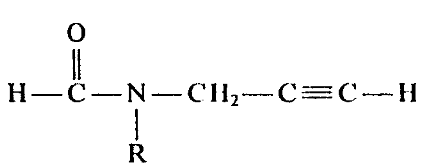

(II)

in welcher
R die oben angegebene Bedeutung hat,

mit Halogenierungsmitteln in Gegenwart von basischen Stoffen in einem Verdünnungsmittel bei Temperaturen zwischen $-30°C$ bis $+50°C$ umsetzt.

Die neuen Halogenpropargylformamide der Formel (I) weisen starke fungizide und bakterizide Eigenschaften auf. Überraschenderweise zeigen sie eine Überlegenheit in ihrer Wirkung gegenüber den vorbekannten Verbindungen auf.

Die erfindungsgemäßen Verbindungen stellen wegen der vielen Möglichkeiten ihrer überlegenen biologischen Anwendung eine wertvolle Bereicherung der Technik dar.

Von den erfindungsgemäßen Halogenpropargylformamiden der Formel (I) sind besonders bevorzugt diejenigen, bei denen

R für Wasserstoff, für Alkyl mit 1 bis 18 Kohlenstoffatomen, wie Methyl, Ethyl, n- und iso-Propyl, n-, sec.-, tert.- und iso-Butyl, Pentyl, Hexyl, 2-Ethylhexyl, Octyl, Undecyl, Dodecyl und Stearyl, für Halogenalkyl mit 1 bis 5 Halogenatomen und 1 bis 4 Kohlenstoffatomen, für Cycloalkyl mit 5 bis 7 Kohlenstoffatomen, wie Cyclopentyl, Cyclohexyl und Cycloheptyl, für Alkenyl mit 2 bis 4 Kohlenstoffatomen, wie Vinyl, Propenyl oder Butenyl, für Alkinyl mit 3 oder 4 Kohlenstoffatomen, wie Propinyl oder Butinyl, für Halogenalkinyl mit 1 oder 2 Halogen- und 3 bis 5 Kohlenstoffatomen, für gegebenenfalls substituiertes Aralkyl mit 1 oder 2 Kohlenstoffatomen im Alkylteil und 6 Kohlenstoffatomen im Arylteil, wie Benzyl oder Phenethyl, wobei der Arylrest vorzugsweise ein- bis fünffach, gleich oder verschieden durch Alkyl mit 1 bis 4 Kohlenstoffatomen, wie Methyl, Ethyl, n- oder iso-Propyl, n-, sec-, tert.- oder iso-Butyl, und Halogen, wie Fluor, Chlor oder Brom, substituiert sein kann, ferner für gegebenenfalls ein- bis fünffach, gleich oder verschieden substituiertes Phenyl steht. Als Substituenten seien genannt Halogen, wie Fluor, Chlor, Brom und Jod, Nitro, Alkyl mit 1 bis 4 Kohlenstoffatomen, Alkoxy mit 1 bis 4 Kohlenstoffatomen, Halogenalkyl mit 1 bis 5 Halogenatomen und 1 bis 4 Kohlenstoffatomen, wie Trifluormethyl, Trichlormethyl, Trichlorethyl und Halogenalkylthio mit 1 bis 5 Halogen- und 1 bis 4 Kohlenstoffatomen und
Hal für Jod und Brom stehen.

Ganz besonders bevorzugt sind die Verbindungen der Formel (I), in welcher

R für Wasserstoff, Alkyl mit 1 bis 18 Kohlenstoffatomen, gegebenenfalls ein- bis dreifach, gleich oder verschieden durch Methyl, Ethyl und Chlor substituiertes Benzyl und Phenethyl, für gegebenenfalls ein- oder dreifach, gleich oder verschieden durch Nitro, Methyl, Ethyl, n- und iso-Propyl, Trifluormethyl, Methoxy, Ethoxy, n-Propoxy, iso-Propoxy, Trifluormethylthio und Trichlormethylthio substituiertes Phenyl und
Hal für Jod und Brom stehen.

Im einzelnen seien beispielsweise außer den Herstellungsbeispielen die folgenden Halogenpropargylformamide genannt:

Brompropargylformamid,
Jodpropargylmethylformamid,
Jodpropargyloctylformamid,
Jodpropargyldodecylformamid,
Jodpropargylformanilid,
2-Chlor-jodpropargylformanilid,
3-Chlor-jodpropargylformanilid,
4-Chlor-jodpropargylformanilid,
2,3-Dichlor-jodpropargylformanilid,
2,4-Dichlor-jodpropargylformanilid,
3,4-Dichlor-jodpropargylformanilid,
3,5-Dichlor-jodpropargylformanilid,
2,4,6-Trichlor-jodpropargylformanilid,
2-Methyl-jodpropargylformanilid,
3-Methyl-jodpropargylformanilid,
4-Methyl-jodpropargylformanilid,
2,3-Dimethyl-jodpropargylformanilid,
2,6-Dimethyl-jodpropargylformanilid,
2-Methyl-6-ethyl-jodpropargylformanilid,

3-Trifluormethyl-jodpropargylformanilid,
3,5-Bis-trifluormethyl-jodpropargylformanilid,
3-Nitro-jodpropargylformanilid,
Brompropargylformanilid,
3-Chlor-brompropargylformanilid.

Der Reaktionsverlauf beim erfindungsgemäßen Verfahren bei Verwendung beispielsweise von Propargylformamid und Jod als Ausgangsmaterialien kann durch das folgende Formelschema wiedergegeben werden:

$$H-\overset{\overset{\displaystyle O}{\|}}{C}-NH-CH_2-C\equiv C-H + J_2 \xrightarrow[\substack{-NaJ \\ -H_2O}]{NaOH} H-\overset{\overset{\displaystyle O}{\|}}{C}-NH-CH_2-C\equiv C-J$$

Die bei der Durchführung des Verfahrens als Ausgangsstoffe einzusetzenden Propargylformamide sind durch die Formel (II) definiert. Die Verbindungen sind größtenteils bekannt oder können nach bekannten Verfahren hergestellt werden, z. B. durch Umsetzung eines entsprechenden Formamids mit z. B. Kalium-tert.-butylat zum Formamid-Kaliumsalz und dieses wird mit einem Propargylhalogenid umgesetzt. Man kann aber auch ein primäres Amin in Gegenwart einer Base mit einem Propargylhalogenid umsetzen und das gebildete Propargylamin mit Ameisensäure oder einem Ameisensäureester formulieren (vgl. z. B. Bull.Soc. Chim. Fr. 1967 (2), Seiten 588—596).

Die weiterhin als Ausgangsmaterialien zu verwendenden Halogenierungsmittel, wie Halogen, sind käufliche leicht zugängliche Produkte.

Als Verdünnungsmittel kommen bei dem Verfahren Wasser und polare organische Lösungsmittel in Frage. Als polare organische Lösungsmittel kommen solche in Frage, die unter den Reaktionsbedingungen inert sind. Es ist vorteilhaft solche organischen Lösungsmittel zu verwenden, die ein gewisses Lösungsvermögen für die Reaktionspartner aufweisen. Vorzugsweise werden Hydroxylgruppen-haltige Lösungsmittel wie z. B. Alkohole verwendet. Als Alkohole kommen vor allem die niederen aliphatischen Vertreter wie z. B. Methanol, Ethanol, Isopropanol aber auch Di- und Polyhydroxyverbindungen, wie Ethylenglykol und Polyethylenglykol in Frage. Besonders bevorzugt wird in Methanol und Wasser gearbeitet.

Als basische Stoffe kommen Alkali- und Erdalkalihydroxide in Frage, bevorzugt werden jedoch Alkalihydroxide, wie z. B. Natrium- oder Kaliumhydroxid verwendet.

Als Halogenierungsmittel kommen elementares Halogen und Hypohalogenite in Frage. Die Hypohalogenite bilden sich bei Zugabe von Halogen zu den oben genannten alkalischen Lösungen, können aber auch vor der Halogenierungsreaktion aus Halogen und Alkalien hergestellt und dann dem zu halogenierenden Material zugegeben werden. Genannt seien vor allem Jod und Brom.

Die Reaktionstemperatur kann —30° bis +50°C betragen, bevorzugt wird jedoch im Bereich von 0—20°C gearbeitet.

Das erfindungsgemäße Verfahren kann beispielsweise wie folgt ausgeführt werden:

Ein Propargylformamid der Formel (II) wird in einem der angegebenen Verdünnungsmittel vorgelegt, die molare bis dreifach molare Menge Alkali, vorzugsweise die 1,5-fache Menge, zugegeben. Anschließend wird portionsweise die mindestens molare Menge Halogenierungsmittel, ein Überschuß desselben bringt keine Nachteile, zugegeben und im Temperaturbereich von 0 bis 20°C nachgerührt. Die Umsetzung wird mit Dünnschichtchromatographie verfolgt. Die Aufarbeitung nach vervollständigter Reaktion erfolgt nach üblichen Methoden, z. B. durch Ausfällen des Endproduktes nach Zugabe von Wasser oder durch Extraktion.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Leptosphaeria nodorum und Fusarium nivale im Getreide, außerdem gegen Venturia am Apfel und gegen Xanthomonas oryzae im Reis eingesetzt werden. Weiterhin sind die breite fungizide Wirkung im Agarplattentest zu nennen. Bei entsprechender Dosierung sind auch akarizide Wirkungen zu verzeichnen.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. Calcit, Marmor, Bims, Sepiolith, Dolomit sowie synthetische Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykolether, Alkylsulfonate, Alkylsulfate, Arylsulfonate sowie Eiweißhydrolysate; als Dispergiermittel kommen in Frage: z. B. Lignin-Sulfitablaufen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akarziziden, Nematiziden, Herbiziden, Schutzstoffen gegen Vogelfraß, Wuchsstoffen, Pflanzennährstoffen und Bodenstrukturverbesserungsmittel.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einen größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Die erfindungsgemäßen Verbindungen eignen sich auch zum Schutz technischer Materialien.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die von Mikroorganismen befallen und/oder zersetzt werden können.

Technische Materialien, die durch die erfindungsgemäßen Substanzen vor einer mikrobiellen Veränderung und Zerstörung geschützt werden sollen, sind beispielsweise Klebstoffe, Leime, Papiere und Kartone, Textilien, Leder, Holz, Anstrichmittel, Putze, Kühlschmiermittel, Dichtungsmassen und Kunststoffartikel, die von Mikroorganismen befallen oder zersetzt werden können. Im Rahmen der zu schützenden Materialien seien auch Teile von Produktionsanlagen, wie beispielsweise Kühlwasser- und Kühlschmiermittelkreisläufe, genannt, deren Funktionstüchtigkeit durch Mikroorganismen beeinträchtigt werden kann. Vorzugsweise können die erfindungsgemäßen Wirkstoffe zum Schutz von Klebstoffen, Papier, Karton, Anstrichfilmen, Holz u. ä. verwendet werden.

Mikroorganismen, die einen Abbau oder eine Veränderung der technischen Materialien bewirken können, sind beispielsweise Bakterien, Pilze, Hefen, Algen und Schleimeorganismen. Vorzugsweise haben die erfindungsgemäßen Substanzen eine starke und breite Wirkung gegen Pilze; von der funkgiziden Wirkung werden Schimmelpilze ebenso erfaßt wie Holz-zerstörende und Holz-verfärbende Pilze.

Es seien beispielsweise Mikroorganismen der folgenden Gattungen genannt:

Alternaria, wie Alternaria tenuis,
Aspergillus, wie Aspergillus niger,
Aureobasidium, wie Aureobasidium pullulans
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Sclerophoma, wie Sclerophoma pityophila,
Trichoderma, wie Trichoderma viride.

Je nach ihrem Anwendungsgebiet können die erfindungsgemäßen Substanzen in die üblichen Formulierungen überführt werden, wie Lösungen, Emulsionen, Suspensionen, Pulver, Pasten und Granulate. Diese können in an sich bekannter Weise hergestellt werden, z. B. durch Vermischen der Wirkstoffe mit einem Streckmittel, das aus flüssigem Lösungsmittel und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Verstreckmitteln, gegebenenfalls organische Solventien, als Hilfslösungsmittel verwendet werden können.

Organische Solventien für die Wirkstoffe können beispielsweise Alkohole, wie niedere Alkohole, vorzugsweise Ethanol oder Isopropanol, oder Benzylalkohol, Ketone wie Aceton oder Methylethylketon, flüssige Kohlenwasserstoffe, wie Benzinfraktionen, chlorierte Kohlenwasserstoffe, wie 1,2-Dichlorethan sein.

Die erfindungsgemäßen mikrobiziden Mittel enthalten im allgemeinen 10 bis 100 Gew.-%, bevorzugt 50 bis 80 Gew.-%, der Halogenpropargylformamide als Wirkstoff.

Die Anwendungskonzentration der erfindungsgemäßen Substanzen richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,01 bis 1 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt: Benzimidazolyl-carbamate, Trihalogenmethylthio-Verbindungen, wie N-Fluordichlormethylthio-phthalimid und N,N-Dimethyl-N'-phenyl-N'-(fluordichlormethylthio)-sulfamid, formaldehydabspaltende Verbindungen, wie Hemiformale, Phenolderivate, wie p-Chlor-m-kresol, 2-Phenylphenol, (2,2'-Dihydroxy-5,5'-dichlor)-diphenylmethan, Dithiocarbamate, Thiazolylbenzimidazol, Isothiazolon- und Benzisothiazolon-Derivate, Tetrachlorisophthalsäuredinitril, Mercaptobenzthiazol, Mercaptopyridin.

## Herstellungsbeispiele

### Beispiel 1

a) 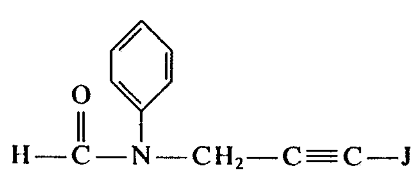

32 g (0,2 Mol) Propargylformanilid werden in 600 ml Methanol gelöst und bei 0—5° C eine Lösung von 10 g (0,25 Mol) Natriumhydroxid 30 ml Wasser zugegeben. Dann werden bei 0° C innerhalb einer Stunde 51 g (0,2 Mol) Jod in mehreren Portionen dosiert und 4 Stunden bei 0° C nachgerührt. Ein Dünnschichtchromatogramm zeigt nun, daß das Ausgangsprodukt vollständig umgesetzt ist. Zur Aufarbeitung wird der Ansatz in ein Gemisch von 2 l Eiswasser und 10 ml 40%iger Natriumhydrogensulfitlösung (zur Entfärbung von Restmengen Jod) eingerührt, wobei nach einiger Zeit Kristallisation erfolgt. Es wird abgesaugt, mit Wasser gewaschen und getrocknet. Die Ausbeute beträgt 44 g (77% der Theorie) beigefarbener Kristalle an N-Jodpropargyl-N-formylanilid, das nach Umkristallisieren aus Ethanol bei 78° C schmilzt.

Das in Beispiel 1 verwendete Ausgangsprodukt wird auf folgende Weise hergestellt:

b) 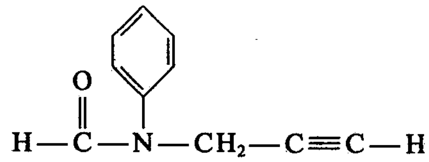

97 g (0,8 Mol) Formanilid werden in 400 ml Tetrahydrofuran gelöst und 90 g (0,8 Mol) Kalium-tertiärbutylat in 400 ml Tetrahydrofuran zugegeben. Nach Abklingen der exothermen Reaktion (Salzbildung) wird am Rotavapor zur Trockene eingeengt. Der Rückstand wird portionsweise bei 0—5°C in eine Lösung von 60 g (0,8 Mol) Propargylchlorid in 500 ml Dimethylformamid eingetragen, dann wird langsam auf Raumtemperatur erwärmt und noch 20 Stunden nachgerührt, bis nach Dünnschichtchromatogramm vollständige Umsetzung erfolgt ist. Der Ansatz wird eingeengt, mit Methylenchlorid-Wasser ausgeschüttelt, die organische Phase abgetrennt, eingeengt und im Vakuum destilliert. Die Fraktion im Bereich 105—120°C/1,5 mm ist Propargylformanilid. Die Ausbeute beträgt 96 g (75% der Theorie). Das Produkt kann ohne weitere Reinigung zur Jodierung eingesetzt werden.

Beispiel 2

a) 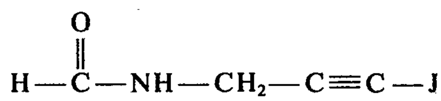

42 g (0,5 Mol) Propargylformamid werden in 1 l Methanol gelöst, bei 0°C eine Lösung von 25 g (0,6 Mol) Natriumhydroxid in 80 ml Wasser und bei gleicher Temperatur 130 g (0,5 Mol) Jod portionsweise zugegeben. Dann wird noch 4 Stunden bei 0°C nachgerührt, der Ansatz in eine Lösung von 5 ml 40%iger Natriumhydrogensulfitlösung in 2 l Wasser eingerührt, 3mal mit je 300 ml Methylenchlorid ausgeschüttelt, die organische Phase abgetrennt, über Natriumsulfat getrocknet und eingeengt. Die Rohausbeute von 45 g wird an einer Kieselgelsäule mit Ethylacetat als Fließmittel chromatographiert. Dabei werden 31 g (57% der Theorie) helle Kristalle an Jodpropargylformamid gewonnen, die nach Umkristallisieren aus Wasser bei 86—88°C schmelzen.

Das in Beispiel 2 verwendete Propargylformamid wird auf folgende Weise hergestellt:

b) 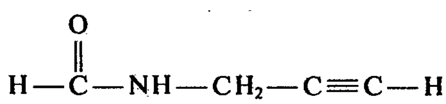

45 g (1 Mol) frisch destilliertes Formamid werden in 600 ml Dimethylformamid vorgelegt, 172 g (1,25 Mol) Kaliumcarbonat und 186 g (2,5 Mol) Chlorpropin zugegeben und erhitzt. Die bei etwa 80—90°C einsetzende exotherme Reaktion wird durch leichte Kühlung auf 100°C gehalten, dann wird noch 15 Stunden bei 100°C nachgerührt. Der Ansatz wird nun am Rotavapor eingeengt und der Rückstand durch Säulenchromatographie an Kieselgel mit Ethylacetat als Fließmittel fraktioniert. Es werden 42 g (56% der Theorie) einer hellen Flüssigkeit erhalten, die bei 120—125°C/20 mbar destilliert werden kann.

Analog dem oben beschriebenen Herstellungsbeispiel 1 bzw. 2 können die nachfolgend beschriebenen Halogenpropargylformamide der Formel (I)

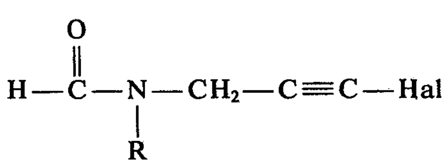

(I)

hergestellt werden:

| Beispiel Nr. | R | Hal | Schmelzpunkt [°C] | Ausbeute [% der Theorie] |
|---|---|---|---|---|
| 3) | 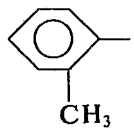 | J | 70 – 80 | 55 |
| 4) | 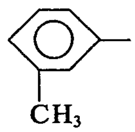 | J | 83 – 84 | 52 |
| 5) | 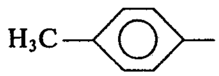 | J | 84 – 87 | 79 |
| 6) | 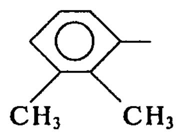 | J | 85 – 90 | 61 |
| 7) | 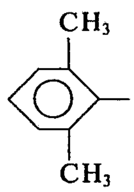 | J | 88 – 90 | 92 |
| 8) | 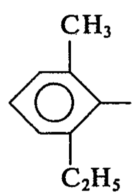 | J | 91 – 97 | 31 |
| 9) | 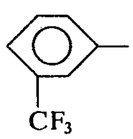 | J | 107 – 109 | 48 |
| 10) | 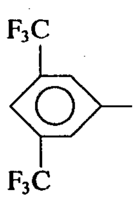 | J | 86 – 90 | 8 |
| 11) | 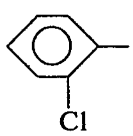 | J | 114 – 119 | 86 |
| 12) | 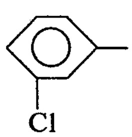 | J | 78 – 81 | 56 |

Fortsetzung

| Beispiel Nr. | R | Hal | Schmelzpunkt [°C] | Ausbeute [% der Theorie] |
|---|---|---|---|---|
| 13) | 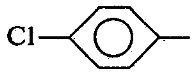 | J | 88 – 89 | 32 |
| 14) | 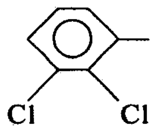 | J | 78 – 81 | 48 |
| 15) | 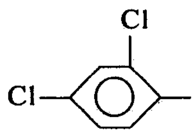 | J | 79 – 81 | 33 |
| 16) | 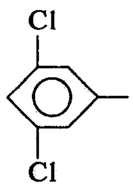 | J | 98 – 102 | 43 |
| 17) | 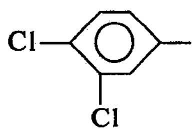 | J | 104 – 106 | 27 |
| 18) | 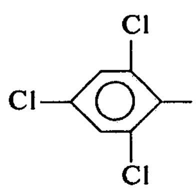 | J | 82 – 84 | 36 |
| 19) | 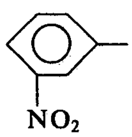 | J | 117 – 119 | 16 |
| 20) | 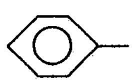 | Br | | 85 |
| 21) | 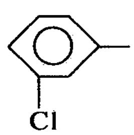 | Br | | 63 |

Die als Ausgangsverbindungen dienenden Propargylformamide der Formel (II) wurden analog den Beispielen 1b) und 2b) hergestellt:

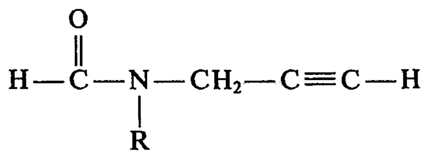 (II)

| R | Schmelzpunkt [°C]; Siedepunkt [°C/mbar], Brechungsindex [$n_D^{20}$] | Ausbeute [% der Theorie] |
|---|---|---|
| 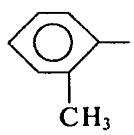 | 43 – 45 | 69 |
| 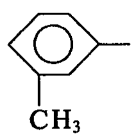 | 1,5470 | 69 |
| 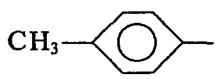 | 158/12 | 58 |
| 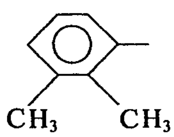 | 52 – 54 | 82 |
| 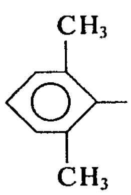 | 57 – 59 | 32 |
| 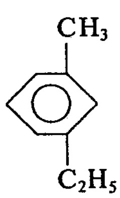 | 1,5020 | 98 |
| 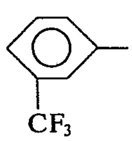 | 1,5328 | 52 |
| 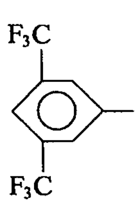 | 52 – 54 | 75 |

Fortsetzung

| R | Schmelzpunkt [°C]; Siedepunkt [°C/mbar], Brechungsindex [$n_D^{20}$] | Ausbeute [% der Theorie] |
|---|---|---|
| 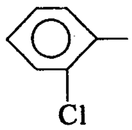 | 107 – 8/0,8 | 63 |
| 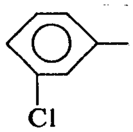 | 44 – 46 | 28 |
| 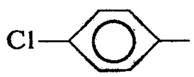 | 51 – 53 | 81 |
| 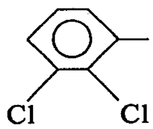 | 72 – 74 | 96 |
| 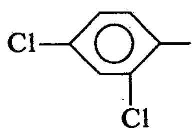 | 71 – 73 | 77 |
| 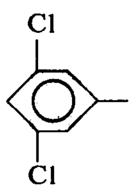 | 77 – 80 | 68 |
| 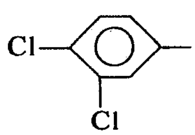 | 69 – 71 | 66 |
| 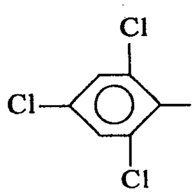 | 70 – 72 | 86 |
| 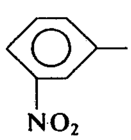 | 82 – 83 | 68 |
| 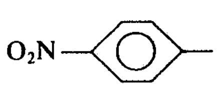 | 114 – 16 | 31 |

In den nachfolgenden Beispielen werden nachstehend angegebene bekannte Verbindungen als Vergleichssubstanzen eingesetzt:

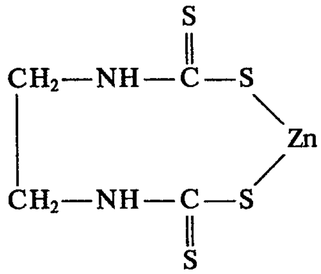

(A)

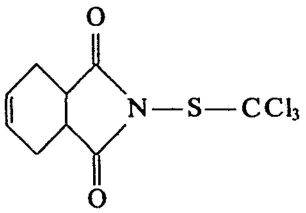

(B)

$3 \times Cu(OH)_2 \times CuCl_2 \times H_2O$ (C)

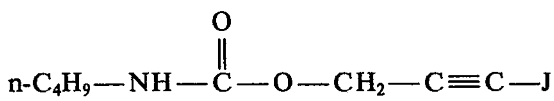

(D)

Beispiel A

Leptosphaeria nodorum-Test (Weizen)/protektiv

Lösungsmittel:
100 Gewichtsteile Dimethylformamid
Emulgator:
0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele:
5, 7, 6, 9, 12, 13, 14, 15 und 16.

Beispiel B

Fusarium nivale-Test (Roggen)/Saatgutbehandlung

Die Anwendung der Wirkstoffe erfolgt als Trockenbeizmittel. Sie werden zubereitet durch Abstrekken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10°C und einer relativen Luftfeuchtigkeit von ca. 95% in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigen bei diesem Test z. B. die Verbindungen gemäß folgender Herstellungsbeispiele: 5 und 9.

## Beispiel C

### Venturia-Test (Apfel)/protektiv

Lösungsmittel:
4,7 Gewichtsteile Aceton
Emulgator:
0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100% relativer Luftfeuchtigkeit in einer Inkubationskabine.

Die Pflanzen werden dann im Gewächshaus bei 20° C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 15, 13 und 17.

## Beispiel D

### Xanthomonas oryzae-Test/Bakteriose/Reis/systemisch

Lösungsmittel:
121,25 Gewichtsteile Aceton
Emulgator:
3,75 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften werden 100 ml der Wirkstoffzubereitung auf Einheitserde gegossen, in der junge Pflanzen angezogen wurden. 3 Tage nach der Behandlung werden die Pflanzen mit einer wäßrigen Suspension von Xanthomonas oryzae durch Stichverletzung inokuliert. Danach verbleiben die Pflanzen 14 Tage bis zur Auswertung in einem Gewächshaus bei 24 bis 26° C und 70 bis 80% rel. Luftfeuchtigkeit.

Eine deutliche Überlegenheit in der Wirksamkeit gegenüber dem Stand der Technik zeigen bei diesem Test z. B. die Verbindungen gemäß folgender Herstellungsbeispiele: 1, 16, 12 und 9.

## Beispiel E

### Wirkung gegen Pilze

In einem Agar, der aus Bierwürze und Pepton hergestellt wurde, wurden die erfindungsgemäßen Verbindungen in abgestuften Konzentrationen zwischen 1 und 5000 mg/l Versuchsprobe eingearbeitet. Nach dem Erstarren des Agars erfolgte die Kontamination der so hergestellten Agarproben mit Reinkulturen verschiedener Testpilze.

Nach zweiwöchiger Lagerung bei 28° C und 60 bis 70% relativer Luftfeuchtigkeit wurde ausgewertet. Es wurde die minimale Hemmkonzentration (MHK), d. h. die geringste in einer Agarprobe enthaltene Konzentration der Substanz angegeben, bei der keinerlei Bewuchs durch die verwendete Art erfolgte.

Eine deutliche Überlegenheit in der Wirksamkeit gegenüber dem Stand der Technik zeigen in diesem Test z. B. die Verbindungen gemäß folgender Herstellungsbeispiele: 11, 12, 13, 15, 17, 18, 5, 7, 14, 3, 4, 6 und 19.

**Patentansprüche**

1. Halogenpropargylformamide der Formel (I)

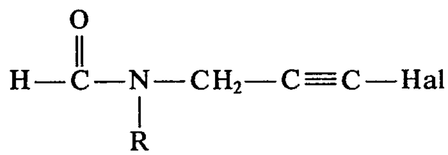

(I)

in welcher

R für Wasserstoff, Alkyl mit 1 bis 20 Kohlenstoffatomen, Halogenalkyl mit 1 bis 5 Halogenatomen und 1 bis 8 Kohlenstoffatomen, für Cycloalkyl mit 5 bis 10 Kohlenstoffatomen, für Alkenyl mit 2 bis 6 Kohlenstoffatomen, für Alkinyl mit 2 bis 6 Kohlenstoffatomen, für Halogenalkinyl mit 1 bis 3 Halogen- und 2 bis 6 Kohlenstoffatomen, für gegebenenfalls durch Alkyl mit 1 bis 6 Kohlenstoffatomen und Halogen gleich oder verschieden, ein- bis fünffach im Arylteil substituiertes Aralkyl mit 1 bis 6 Kohlenstoffatomen im Alkylteil und 6 bis 10 Kohlenstoffatomen im Arylteil, für gegebenenfalls ein- bis fünffach, gleich oder verschieden durch Alkyl mit 1 bis 6 Kohlenstoffatomen, Nitro, Halogen, Alkoxy mit 1 bis 6 Kohlenstoffatomen, Halogenalkyl mit 1 bis 5 Halogen- und 1 bis 6 Kohlenstoffatomen, und Halogenalkylthio mit 1 bis 5 Halogen- und 1 bis 6 Kohlenstoffatomen, und Halogenalkylthio mit 1 bis 5 Halogen- und 1 bis 6 Kohlenstoffatomen substituiertes Aryl und
Hal für Jod oder Brom stehen.

2. Halogenpropargylformamide gemäß Formel (I) in Anspruch 1, wobei

R für Wasserstoff, Alkyl mit 1 bis 18 Kohlenstoffatomen, gegebenenfalls ein- bis dreifach, gleich oder verschieden durch Methyl, Ethyl und Chlor substituiertes Benzyl und Phenethyl, für gegebenenfalls ein- bis dreifach, gleich oder verschieden durch Nitro, Methyl, Ethyl, n- und iso-Propyl-, Trifluormethyl, Methoxy, Ethoxy, n-Propoxy, iso-Propoxy, Trifluormethylthio und Trichlormethylthio substituiertes Phenyl und
Hal für Jod und Brom stehen.

3. Verfahren zur Herstellung von Halogenpropargylformamiden der Formel (I)

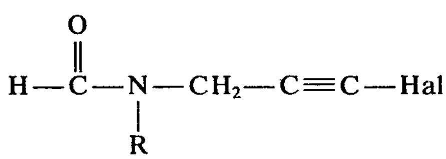

(I)

in welcher

R für Wasserstoff, Alkyl mit 1 bis 20 Kohlenstoffatomen, Halogenalkyl mit 1 bis 5 Halogenatomen und 1 bis 8 Kohlenstoffatomen, für Cycloalkyl mit 5 bis 10 Kohlenstoffatomen, für Alkenyl mit 2 bis 6 Kohlenstoffatomen, für Alkinyl mit 2 bis 6 Kohlenstoffatomen, für Halogenalkinyl mit 1 bis 3 Halogen- und 2 bis 6 Kohlenstoffatomen, für gegebenenfalls durch Alkyl mit 1 bis 6 Kohlenstoffatomen und Halogen gleich oder verschieden, ein- bis fünffach im Arylteil substituiertes Aralkyl mit 1 bis 6 Kohlenstoffatomen im Alkylrest und 6 bis 10 Kohlenstoffatomen im Arylrest, für gegebenenfalls ein- bis fünffach, gleich oder verschieden durch Alkyl mit 1 bis 6 Kohlenstoffatomen, Halogen, Nitro, Alkoxy mit 1 bis 6 Kohlenstoffatomen, Halogenalkyl mit 1 bis 5 Halogen- und 1 bis 6 Kohlenstoffatomen, und Halogenalkylthio mit 1 bis 5 Halogen- und 1 bis 6 Kohlenstoffatomen substituiertes Aryl und
Hal für Jod oder Brom stehen,

dadurch gekennzeichnet, daß man Propargylformamide der Formel (II)

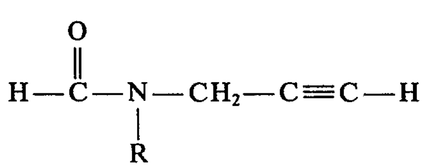

(II)

in welcher

R die angegebene Bedeutung hat,

mit Halogenierungsmitteln in Gegenwart von basischen Stoffen in einem Verdünnungsmittel bei Temperaturen zwischen —30 bis 50° C umsetzt.

4. Schädlingsbekämpfungsmittel, gekennzeichnet durch einen Gehalt mindestens einem Halogenpropargylformamid der Formel (I).

5. Verwendung von Halogenpropargylformamiden der Formel (I) zur Bekämpfung von Schädlingen.

6. Verfahren zur Bekämpfung von Schädlingen, dadurch gekennzeichnet, daß man Halogenpropargylformamide der Formel (I) auf Schädlinge und/oder ihren Lebensraum einwirken läßt.

7. Verfahren zur Herstellung von Schädlingsbekämpfungsmitteln, dadurch gekennzeichnet, daß man Halogenpropargylformamide der Formel (I) mit Streckmitteln und/oder oberflächenaktiven Mitteln vermischt.

8. Mikrobizide Mittel zum Schutz technischer Materialien, gekennzeichnet durch einen Gehalt von mindestens einem Halogenpropargylformamid der Formel (I) gemäß Ansprüchen 1 und 3.

9. Verwendung von Halogenpropargylformamiden der Formel (I) gemäß Ansprüchen 1 und 3 zum Schutz technischer Materialien.

**Claims**

1. Halogenopropargylformamides of the formula (I)

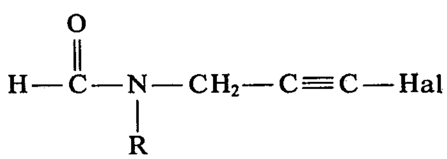

(I)

in which

R represents hydrogen, alkyl with 1 to 20 carbon atoms, halogenoalkyl with 1 to 5 halogen atoms and 1 to 8 carbon atoms, cycloalkyl with 5 to 10 carbon atoms, alkenyl with 2 to 6 carbon atoms, alkinyl with 2 to 6 carbon atoms, halogenoalkinyl with 1 to 3 halogen and 2 to 6 carbon atoms, aralkyl which has 1 to 6 carbon atoms in the alkyl part and 6 to 10 carbon atoms in the aryl part and is optionally mono- to pentasubstituted in the aryl part by identical or different substituents from amongst alkyl with 1 to 6 carbon atoms and halogen, or aryl which is optionally mono- to pentasubstituted by identical or different substituents from amongst alkyl with 1 to 6 carbon atoms, nitro, halogen, alkoxy with 1 to 6 carbon atoms, halogenoalkyl with 1 to 5 halogen and 1 to 6 carbon atoms, and halogenoalkylthio with 1 to 5 halogen and 1 to 6 carbon atoms, and

Hal represents iodine or bromine.

2. Halogenopropargylformamides according to formula (I) in Claim 1, wherein

R represents hydrogen, alkyl with 1 to 18 carbon atoms, phenethyl and benzyl optionally mono- to trisubstituted by identical or different substituents from amongst methyl, ethyl and chlorine, or phenyl which is optionally mono- to trisubstituted by identical or different substituents from amongst nitro, methyl, ethyl, n- and iso-propyl-, trifluoromethyl, methoxy, ethoxy, n-propoxy, iso-propoxy, trifluoromethylthio and trichloromethylthio, and

Hal represents iodine and bromine.

3. Process for the preparation of halogenopropargylformamides of the formula (I)

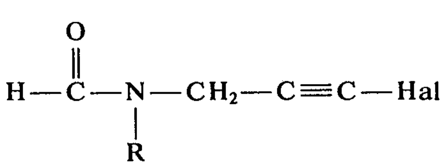

(I)

in which

R represents hydrogen, alkyl with 1 to 20 carbon atoms, halogenoalkyl with 1 to 5 halogen atoms and 1 to 8 carbon atoms, cycloalkyl with 5 to 10 carbon atoms, alkenyl with 2 to 6 carbon atoms, alkinyl with 2 to 6 carbon atoms, halogenoalkinyl with 1 to 3 halogen and 2 to 6 carbon atoms, aralkyl

which has 1 to 6 carbon atoms in the alkyl radical and 6 to 10 carbon atoms in the aryl radical and is optionally mono- to pentasubstituted in the aryl part by identical or different substituents from amongst alkyl with 1 to 6 carbon atoms and halogen, or aryl which is optionally mono- to pentasubstituted by identical or different substituents from amongst alkyl with 1 to 6 carbon atoms, halogen, nitro, alkoxy with 1 to 6 carbon atoms, halogenoalkyl with 1 to 5 halogen and 1 to 6 carbon atoms, and halogenoalkylthio with 1 to 5 halogen and 1 to 6 carbon atoms, and

Hal represents iodine or bromine,

characterised in that propargylformamides of the formula (II)

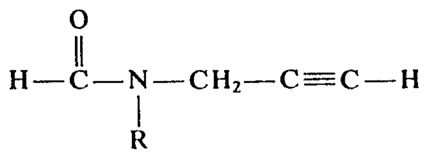

(II)

in which

R has the meaning given,

are reacted with halogenating agents in the presence of basic substances in a diluent at temperatures between −30 and 50°C.

4. Pest-combating agents, characterised in that they contain at least one halogenopropargylformamide of the formula (I).

5. Use of halogenopropargylformamides of the formula (I) for combating pests.

6. Method of combating pests, characterised in that halogenopropargylformamides of the formula (I) are allowed to act on pest and/or their habitats.

7. Process for the preparation of pest-combating agents, characterised in that halogenopropargylformamides of the formula (I) are mixed with extenders and/or surface-active agents.

8. Microbicidal agents for the protection of industrial materials, characterised in that they contain at least one halogenopropargylformamide of the formula (I) according to Claims 1 and 3.

9. Use of halogenopropargylformamides of the formula (I) according to Claims 1 and 3 for the protection of industrial materials.

**Revendications**

1. Halogénopropargylformamides de formule (I):

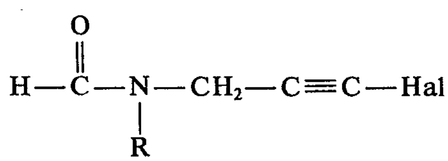

(I)

dans laquelle

R représente un atome d'hydrogène, un groupe alkyle ayant 1 à 20 atomes de carbone, halogénoalkyle ayant 1 à 5 atomes d'halogène et 1 à 8 atomes de carbone, cycloalkyle ayant 5 à 10 atomes de carbone, alcényle ayant 2 à 6 atomes de carbone, alcynyle ayant 2 à 6 atomes de carbone, halogénoalcynyle ayant 1 à 3 atomes d'halogène et 2 à 6 atomes de carbone, aralkyle (ayant 1 à 6 atomes de carbone dans la partie alkyle et 6 à 10 atomes de carbone dans la partie aryle, éventuellement substitué 1 à 5 fois, de façon identique ou différente, dans la partie aryle par un groupe alkyle ayant 1 à 6 atomes de carbone et par de l'halogène), un groupe aryle (éventuellement substitué 1 à 5 fois, de façon identique ou différente, par un groupe alkyle ayant 1 à 6 atomes de carbone, nitro, halogéno, alcoxy ayant 1 à 6 atomes de carbone, halogénoalkyle ayant 1 à 5 atomes d'halogène et 1 à 6 atomes de carbone, et halogénoalkylthio ayant 1 à 5 atomes d'halogène et 1 à 6 atomes de carbone),et

Hal représente de l'iode ou du brome.

2. Halogénopropargylformamides selon la formule (I) de la revendication 1, dans lesquels:

R représente un atome d'hydrogène, un groupe alkyle ayant 1 à 18 atomes de carbone, un groupe benzyle et phénéthyle (évetuellement substitué 1 à 3 fois, de façon identique ou différente, par un groupe méthyle, éthyle et par du chlore), un groupe phényle (éventuellement substitué 1 à 3 fois,

de manière indentique ou différente, par un groupe nitro, méthyle, éthyle, n- et iso-propyle, trifluorométhyle, méthoxy, éthoxy, n-propoxy, iso-propoxy, trifluorométhylthio et trichlorométhylthio), et

Hal représente de l'iode et du brome.

3. Procédé de préparation d'halogénopropargylformamides de formule (I):

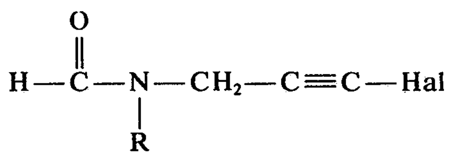

(I)

dans laquelle

R représente un atome d'hydrogène, un groupe alkyle ayant 1 à 20 atomes de carbone, halogénoalkyle ayant 1 à 5 atomes d'halogène et 1 à 8 atomes de carbone, cycloalkyle ayant 5 à 10 atomes de carbone, alcényle ayant 2 à 6 atomes de carbone, alcynyle ayant 2 à 6 atomes de carbone, halogénoalcynyle ayant 1 à 3 atomes d'halogène et 2 à 6 atomes de carbone, aralkyle (comportant 1 à 6 atomes de carbone dans le radical alkyle et 6 à 10 atomes de carbone dans le radical aryle, éventuellement substitué 1 à 5 fois dans la partie aryle, de façon identique ou différente, par un groupe alkyle ayant 1 à 6 atomes de carbone et par de l'halogène), un groupe aryle (éventuellement substitué 1 à 5 fois, de façon identique ou différente, par un groupe alkyle ayant 1 à 6 atomes de carbone, halogéno, nitro, alcoxy ayant 1 à 6 atomes de carbone, halogénoalkyle ayant 1 à 5 atomes d'halogène et 1 à 6 atomes de carbone, halogénoalkylthio ayant 1 à 5 atomes d'halogène et 1 à 6 atomes de carbone), et

Hal représente de l'iode ou du brome,

caractérisé en ce qu'on fait réagir des propargylformamides de formule (II):

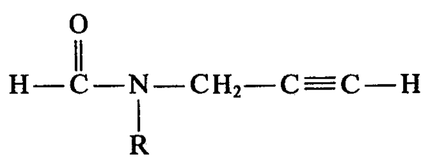

(II)

dans laquelle

R a le sens indiqué,

avec des agents d'halogénation en présence de substances basiques dans un diluant à des températures comprises entre —30 et 50° C.

4. Pesticide, caractérisé par une teneur en au moins un halogénopropargylformamide de formule (I).

5. Utilisation des halogénopropargylformamides de formule (I) pour la lutte contre les parasites.

6. Procédé de lutte contre des parasites, caractérisé en ce qu'on fait agir des halogénopropargylformamides de formule (I) sur les parasites et/ou sur leur biotope.

7. Procédé de préparation de pesticides, caractérisé en ce qu'on mélange des halogénopropargylformamides de formule (I) avec des agents d'allongement et/ou des agents tensioactifs.

8. Produit microbicide destiné à protéger des matières techniques, caractérisé par une teneur en au moins un halogénopropargylformamide de formule (I) selon les revendications 1 et 3.

9. Utilisation d'halogénopropargylformamides de formule (I) selon les revendications 1 et 3 pour la protection de matières techniques.